# EUROPÄISCHE PATENTANMELDUNG

(11) **EP 0 823 485 A1**
(43) Veröffentlichungstag der Anmeldung: **11.02.1998**
(21) Anmeldenummer: 97113449.9
(22) Anmeldetag: 05.08.1997
(51) Int. Cl.: C12Q 1/68

(54) **Verfahren zur Amplifikation von Neisseria gonorrhoeae Nukleinsäuresequenzen**

(30) Priorität: 08.08.1996 DE 19632041
(71) Anmelder: BOEHRINGER MANNHEIM GMBH, 68298 Mannheim (DE)
(72) Erfinder: Wyrich, Ralf, Dr., 68623 Lampertheim (DE); Lichtinghagen, Ralf, Dr., 30173 Hannover (DE)

(57) **Zusammenfassung**

Ein Verfahren zum Nachweis von Neisseria gonorrhoeae durch Amplifikation einer Neisseria gonorrhoeae spezifischen Nukleotidsequenz und Nachweis der Amplifikationsprodukte, bei dem ein Primer mit der N. gonorrhoeae spezifischen Nukleotidsequenz in einem Bereich hybridisieren kann, der Position 1339 der 23S rRNA einschließt ist besonders gut zur Differenzierung von Neisseria gonorrhoeae von Neisseria meningitidis und anderen Erregern geeignet.

## Beschreibung

Gegenstand der Erfindung sind Verfahren zur Amplifikation und Nachweis von Neisseria gonorrhoeae spezifischen Nukleotidsequenzen mit speziellen Primern sowie ein Set zur Durchführung dieser Verfahren.

Die Geschlechtskrankheit Gonorrhoe ist eine der häufigsten meldepflichtigen Infektionskrankheiten der Welt. Daher hat es nicht an Anstrengungen gefehlt, den Erreger dieser Krankheit, Neiserria gonorrhoeae, nachweisen zu können. Besondere Aufmerksamkeit haben hierbei Nachweisverfahren gefunden, die auf dem Nachweis von Nukleotidsequenzen aus diesem Erreger in menschlichen Proben beruhen. So wurde beispielsweise in EP-A-0 272 009 ein Verfahren beschrieben, bei dem als Nukleinsäuresonden Fragmente eingesetzt wurden, deren Sequenzen komplementär zu bestimmten Teilbereichen der rRNA sind. Die hierbei erzielten Testergebnisse sind jedoch nicht eindeutig. In EP-A-0 408 077 sind Oligonukleotidsonden beschrieben, die Teilbereichen der 16S rRNA von Neisseria gonorrhoeae entsprechen. In WO 90/14442 sind Sonden beschrieben, die der 16S rRNA sowie dem 5'-Bereich des 23S rRNA Gens entsprechen. Einige der beschriebenen Sonden sind jedoch nicht für Neisseria gonorrhoeae spezifisch. Dasselbe gilt für die in EP-A-0 337 896 beschriebenen Sonden. In EP-A-0 574 852 sind Sonden beschrieben, die auch 23S rRNA entsprechen.

In EP-A-0 630 971 ist die Amplifikation von Neisseria-Nukleotidsequenzen beschrieben, die im Bereich des Cytosinenmethyltransferase Gens lokalisiert sind.

Aufgabe der vorliegenden Erfindung war es, den Stand der Technik ganz oder teilweise zu verbessern und insbesondere Primer zur Verfügung zu stellen, bei denen eine sehr geringe Primerselbstamplifikation beobachtet wird.

Gegenstand der Erfindung ist ein Verfahren zum Nachweis von Neisseria gonorhoeae durch Amplifikation einer Neisseria gonorrhoeae spezifischen Nukeotidsequenz und Nachweis der Amplifikationsprodukte, wobei die Amplifikation mit Hilfe mindestens eines Primers durchgeführt wird, der mit der Neisseria gonorrhoeae spezifischen Nukleotidsequenz innerhalb der 23S rRNA-Gensequenz, bevorzugt in einem Bereich, der Position 1339 bezogen auf die EMBL Datenbanksequenz Nr. x67293 der 23S rRNA einschließt, hybridisieren kann, oder der mit dem entsprechenden Gegenstrang in diesem Bereich hybridisieren kann.

Unter einem Nachweisverfahren für Neisseria gonorrhoeae wird ein Verfahren verstanden, mit welchem die Anwesenheit, bevorzugt die Menge von Neisseria gonorrhoeae in einer Probe bestimmt wird. Das erfindungsgemäße Verfahren benutzt hierzu eine Probenflüssigkeit, in weicher die Anwesenheit von Neisseria gonorrhoeae vermutet wird oder bestätigt werden soll, Amplifikationen von Neisseria gonorrhoeae spezifischen Nukleotidsequenzen und Bestimmung der Amplifikationsprodukte als Zeichen für die Anwesenheit von Neisseria gonorrhoeae in der ursprünglichen Probe.

Als Probenflüssigkeiten werden bevorzugt Körperflüssigkeiten und davon abgeleitete Flüssigkeiten verwendet. Besonders bevorzugt sind Abstriche von Cervix uteri, Urethra oder Rachen oder Urin.

Wenngleich es erstrebenswert ist, das ursprüngliche Probenmaterial direkt in ein Amplifikationsverfahren einzuführen, hat es sich jedoch als vorteilhaft erwiesen, die Probe in ein- oder mehrstufigen Verfahren vorzubereiten. Diese Vorbereitung kann insbesondere die Freisetzung der Neisseria-Nukleinsäuren aus Neisserien beinhalten. Solche Lyseverfahren sind dem Fachmann bekannt. Ferner ist es möglich, die Nukleotidsequenzen durch spezifische oder unspezifische Affinitätsbindung aufzureinigen und von eventuell störenden weiteren Probenbestandteilen zu befreien. Dies kann beispielsweise geschehen durch Adsorption der Nukleinsäuren an glasartige Oberflächen in Gegenwart von chaotropen Salzen. Im Falle der Affinitätsreinigung werden die aufgereinigten Nukleinsäuren bevorzugt vor der weiteren Bearbeitung in eine flüssige Phase überführt. Solche Verfahren sind dem Fachmann, beispielsweise aus WO 94/10564, bekannt.

Für das erfindungsgemäße Verfahren besonders geeignete Amplifikationen sind beispielsweise die Verfahren, die auf der Polymerasekettenreaktion beruhen, wie z. B. geschildert in EP-A-0 201 184. In diesem Verfahren wird unter Zuhilfenahme eines ersten Primers, der zu einem Strang der nachzuweisenden Nukleinsäure komplementär ist, mit Hilfe einer DNA-Polymerase ein Verlängerungsprodukt des Primers gebildet, wobei der Strang als Matrize für die Inkorporierung von entsprechenden Mondesoxyribonukleosidtriphosphaten dient. Das Verlängerungsprodukt des ersten Primers dient als Matrize für die Verlängerung eines Primers, der zu einem vom ersten Primer aus gesehen in 3'-Richtung gelegenen Bereich des Verlängerungsprodukts komplementär ist. Das in der zweiten Verlängerungsreaktion gebildete Verlängerungsprodukt kann als Matrize für die Verlängerung eines weiteren Moleküls des ersten Primers dienen. Durch mehrfach zyklische Aufeinanderfolge der Schritte Primerhybridisierung, Primerverlängerung und Denaturierung der gebildeten Doppelstränge wird eine theoretisch exponentielle Vermehrung der ursprünglichen Sequenzen erreicht, wobei die einmal gebildeten Verlängerungsprodukte immer wieder als Matrize für die Verlängerung von Primern dienen können. Reagenzien, Geräte und Reaktionsbedingungen sind dem Fachmann auf dem Gebiet der Nukleinsäurediagnostik geläufig.

Ein Kern der vorliegenden Erfindung ist die Wahl eines Primers dergestalt, daß er die Positionen 1339 oder 1502 der 23S rRNA-Gensequenz einschließt oder mit dem entsprechenden Gegenstrang in diesem Bereich hybridisieren kann. Hintergrund für diese Auswahl ist die Tatsache, daß sich gerade diese Positionen erfindungsgemäß für eine Differenzierung zwischen den nahe verwandten Neisseria spezies N. gonorrhoeae und N. meningitidis erfindungsgemäß besonders eignen. Während N. gonorrhoeae (EMBL Datenbank Zugangsnummer x67293) in Position 1339 A als Base aufweist, ist diese Base bei N. meningitidis G (EMBL-Datenbank Zugangsnummer x67300). In Position 1502 findet sich bei N. gonorrhoeae T, während diese Position bei N. meningitidis C aufweist. Dabei ist es für die Erfindung nicht wesentlich, ob die 23S rRNA-Sequenz direkt zum Nachweis herangezogen wird, oder deren Gegenstrang zur Hybridisierung mit dem ersten Primer eingesetzt wird. Wesentlich ist nur, daß der oder die Primer mit der Nukleotidsequenz oder dem Gegenstrang hybridisieren können. Die Hybridisierung muß nicht unbedingt spezifisch sein, wenn ein Primer gewählt wird, dessen 3'-terminale Base komplementär zu der Base in Position 1339 bzw. 1502 ist. In diesem Fall bildet sich bei Hybridisierung des Primers mit Nukleotidsequenzen aus N. meningitidis ein Mismatch am 3'-Ende des Primers, was dazu führt, daß eine Primerverlängerung an diesem Ende im wesentlichen oder vollständig unterbleibt. Dieser Effekt kann durch Wahl von Primern noch weiter unterstützt werden, die in dem 2 bis 8, bevorzugt 2 bis 5 Basen vom 3'-Ende des Primers entfernten Bereich ein oder mehrere weitere Mismatches aufweisen. Die Grundzüge dieses Verfahrens sind in EP-A-0 332 435 beschrieben.

Die Länge und Nukleotidsequenz der im erfindungsgemäßen Verfahren eingesetzten Primer wird im wesentlichen durch die erforderliche Spezifität bestimmt. Ein Fachmann kann aufgrund der bekannten Nukleotidsequenz von Neisseria gonorrhoeae (Nucl. Acids Res. 20 (17), 4657 (1992)) in der Gegend der Positionen 1339 und 1502 entsprechend komplementäre Primersequenzen entwerfen. Wegen der erforderlichen Spezifität hat sich eine Primerlänge von 14 oder mehr Basen als zweckmäßig erwiesen. Bevorzugt ist eine Primerlänge von 17 bis 25 Basen.

Bei den Primern kann es sich prinzipiell um jedes, z. B. durch eine Polymerase, unter Anfügung weiterer Nukleotide verlängerbare Molekül handeln, welches aufgrund von Basen-Basen-Wechselwirkungen mit der spezifischen Nukleotidsequenz binden kann. Es kann sich daher sowohl um unnatürliche Moleküle handeln, wie z. B. Peptidnukleinsäuren mit einem verlängerbaren Ende (wie z. B. beschrieben in WO 95/08556), Ribonukleinsäuren oder Desoxyribonukleinsäuren. Besonders bevorzugt sind Oligodesoxyribonukleotide.

Ein Kern der vorliegenden Erfindung ist im wesentlichen die Tatsache, daß sich Unterschiede in der Basensequenz der 23S rRNA von N. gonorrhoeae und N. meningitidis, insbesondere in Position 1339, besonders gut zur Differenzierung zwischen diesen beiden Neisserienarten eignet. Die Differenzierung ist dann besonders gut, wenn der Primer so gewählt wird, daß die 3'-terminale Base des Primers komplementär oder homolog ist zu der Base in Position 1339 der 23S rRNA von N. gonorrhoeae (nicht jedoch zu der entsprechenden Base von N. meningitidis). Besonders bevorzugt ist diese 3'-terminale Base des Primers A.

Eine weitere Möglichkeit der Erhöhung der Differenzierbarkeit wird dadurch erreicht, daß die Amplifikation unter Zuhilfenahme eines zweiten Primers durchgeführt wird, der eine Differenzierung an Position 1502 der 23S rRNA von N. gonorrhoeae zuläßt. Auch in diesem Falle ist es bevorzugt, wenn die 3'-terminale Base des zweiten Primers komplementär oder homolog ist zu der Base in Position 1502 der 23S rRNA von N. gonorrhoeae (nicht jedoch zu der entsprechenden Base von N. meningitidis). Bevorzugt ist diese Base ebenfalls A.

Noch weiter bevorzugt sind Verfahren, bei denen einer oder besonders bevorzugt beide der Primer in einem 2 bis 5 Basen vom 3'-Ende des Primers entfernten Bereich ein oder mehrere Mismatches zu der spezifischen Nukleotidsequenz oder deren Gegenstrang von N. gonorrhoeae aufweist. Bevorzugt ist Position 4 vom 3'-Ende der Primer.

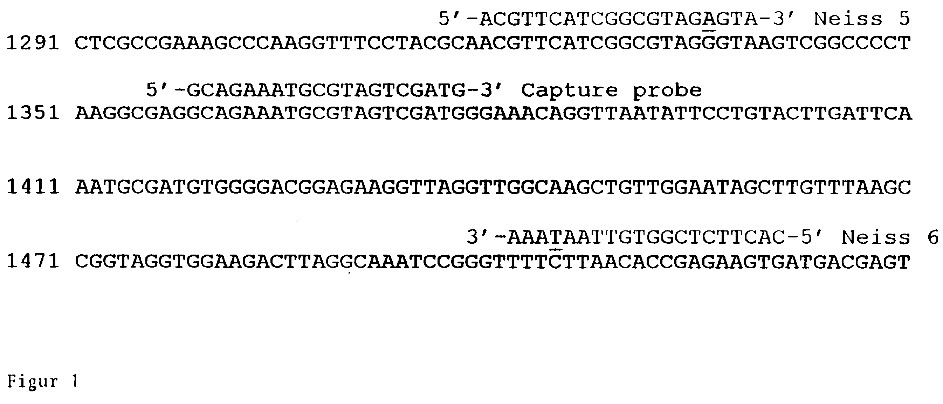

Eine besonders bevorzugte Konstellation von Primern und Sonden ist in FIG 1 gezeigt. Darin ist ein Ausschnitt aus der N. gonorrhoeae Gensequenz für die 23S rRNA dargestellt. Die Zahlen geben die Nukleotidspositionen an. Die unterstrichenen Basen der Primer zeigen die zusätzlich eingeführten Basenaustausche.

Für diesen Fall wird eine hervorragende Differenzierung zwischen N. gonorrhoeae und N. meningitidis erreicht. Es hat sich gezeigt, daß bei Verfahren, die eine Amplifikation beinhalten, die Auswahl der Primerhybridisierungsstellen und die Auswahl der Primersequenzen nicht trivial ist. Es haben sich nämlich hierfür nur bestimmte Positionen bzw. Mismatchanordnungen als für die hohe Differenzierung erforderlich erwiesen. Es ist ein Verdienst der vorliegenden Erfindung, erkannt zu haben, daß eine differenzierende Amplilfikation bei Neisserien und insbesondere im Bereich der 23S rRNA-Sequenzen möglich ist. Um dies zu zeigen, wurde ein Primerpaar entworfen, von dem die Erfinder basierend auf dem Stand der Technik davon ausgegangen wären, daß eine Differenzierung zwischen N. gonorrhoeae und N. meningitidis möglich gewesen wäre. Es hat sich jedoch gezeigt, daß eine ausreichende Differenzierung mit diesem Primerpaar nicht ohne weiteres möglich war. Unter Mismatch wird in diesem Zusammenhang das Vorliegen zweier Basen in einem Doppelstrang verstanden, die in dieser einen Position nicht zueinander komplementär sind.

Die erfindungsgemäßen Primer haben jedoch auch bevorzugt den Vorteil, daß die Bildung von Primeramplifikaten gegenüber anderen Primern stark reduziert ist. Dies gilt insbesondere für Verfahren, bei denen zwei Primer für die Amplifikation eingesetzt werden. Unter Primeramplifikaten werden unspezifische Amplifikationsprodukte verstanden, die sich auch bilden, wenn keine nachzuweisende Nukleotidsequenz in der Probe vorhanden ist. Ursache für solche Primeramplifikate können z. B. die Bildung von Primerdimeren oder die Loopbildung sein. Erfindungsgemäß werden Schwankungen in der Effizienz der Amplifikation geringer Mengen von N. gonorrhoeae DNA, wie z. B. 1000 Zielsequenzen, reduziert.

Es hat sich erfahrungsgemäß gezeigt, daß ein Nachteil der bisher verwendeten Primerpaare darin liegt, daß mit ihnen Nukleinsäuren amplifiziert werden, die als Verunreinigung in einem der Einsatzstoffe, insbesondere der DNA-Polymerase, z. B. Taq-Polymerase, enthalten ist. Die Amplifikation dieser verunreinigenden Nukleinsäuren führt zu ähnlichen Amplifikaten wie die Amplifikation der Nukleotidsequenzen von N. gonorrhoeae. Entsprechende Verunreinigungen sind in Polymerasen verschiedener Hersteller enthalten (Molecular and Cellular Probes 4, 445 - 450 (1990)). Die erfindungsgemäßen Primer führen bevorzugt nicht zu einer Amplifikation dieser Verunreinigungen, die sich in einem Hintergrundsignal bei Wasserproben, d. h. Proben, die anstelle der N. gonorrhoeae nur reines Wasser enthalten, zeigen würden.

Nach der Amplifikation liegen eine große Menge von Amplifikaten, gegebenenfalls neben weiteren Nukleinsäuren der Probenflüssigkeit vor. Die Anwesenheit dieser Amplifikate wird nun zum Gegenstand des Nachweises von N. gonorrhoeae gemacht. Dies kann beispielsweise dadurch geschehen, daß die Amplifikate während der Amplifikation markiert werden und die eingebaute Markierung nun bestimmt wird. Als besonders vorteilhaft haben sich solche Verfahren erwiesen, bei denen die Amplifikate an eine feste Phase gebunden werden und die sie umgebende Flüssigkeit abgetrennt wird. Dies kann beispielsweise durch eine sequenzspezifische Fangsonde, die zu einem Bereich des Amplifikates komplementär ist, geschehen. Die an die feste Phase gebundenen Amplifikate können über eventuell eingebaute Markierungen oder über eine separate Detektionssonde, die wiederum zu einem anderen Bereich desselben Stranges der Amplifikate, zu den die Fangsonde komplementär ist, komplementär ist, geschehen. Diese Verfahren zur Detektion von Hybriden aus Nukleinsäuresträngen, von denen mindestens einer detektierbar markiert ist, sind dem Fachmann bekannt. In der Regel wird die Auswertung apparativ vorgenommen. Hierbei wird angenommen, daß die Signale, welche über einem bestimmten Schwellenwert liegen, von Proben erreicht werden, die N. gonorrhoeae enthielten, während die darunter liegenden Signale nur ein unspezifisches Hintergrundsignal darstellen, eventuell hervorgerufen durch die Anwesenheit ähnlicher Mikroorganismen, z. B. N. meningitidis.

In einer besonders bevorzugten Ausführungsform zum Nachweis von N. gonorrhoeae wird ein Cervix-Abstrich in einem üblichen Transportpuffer aufgenommen und 200 µl dieser Lösung mit 200 µl Phenol/Chloroform (Verhältnis 1:1) und einmal mit Chloroform extrahiert, die DNA mit 0,3 M Natriumacetat und 2,5 Volumen Ethanol gefällt und in 200 µl Wasser rückgelöst. Von der erhaltenen Lösung werden 20 µl mit Primern, Monodesoxynukleosidtriphosphaten, von denen eines mit einer Markierungsgruppe, z. B. Digoxigenin, EP-B-0 324 474) gelabelt ist und einer DNA-Polymerase (z. B. Thermus aquaticus Polymerase) gemischt.

Diese Reaktionsmischung wird Temperaturzyklen unterworfen, bis eine ausreichende Menge von Amplifikaten erzeugt ist. Die Amplifikate werden in einem Analyseautomaten nachgewiesen. Dies geschieht durch Hybridisierung der Amplifikate an eine biotinylierte Fangsonde, die im amplifizierten Bereich hybridisiert und Bindung dieser Sonde an ein Streptavidin beschichtetes Testtube. An die digoxigenierten Amplifikate bindet ein POD-markierter anti Dig-Antikörper, der ein Substrat umsetzt, dessen Farbänderung photometrisch bestimmt wird. Die Absorptionszunahme wird gemessen und bewertet und dient als Maß für die Anwesenheit von N. gonorrhoeae.

Ebenfalls Gegenstand der Erfindung ist ein Set von mindestens 2 Primern, welche eine Nukleotidsequenz aufweisen, die eine spezifische Hybridisierung a) des ersten Primers mit einer Neisseria gonorrhoeae spezifischen Nukleotidsequenz und b) des zweiten Primers mit einem Gegenstrang erlauben, wobei einer der Primer in einem Bereich hybridisiert, der der Sequenz der 23S rRNA entspricht und der insbesondere die Position 1339 der 23S rRNA von Neisseria gonorrhoeae einschließt. Es gelten die oben genannten bevorzugten Ausführungsformen. Dieses Set von Primern liegt bevorzugt als Gemisch dieser mindestens 2 Primer vor, jedoch ist es auch möglich, diese mindestens 2 Primer in getrennten Behältern aufzubewahren. Das erfindungsgemäße Primerset kann auch Bestandteil eines Reagenzkits zur Amplifikation oder/und zum Nachweis von Neisseria gonorrhoeae sein. Dazu enthält das Reagenzkit bevorzugt ein Gemisch von Monodesoxyribonukleosidtriphosphaten, von denen eine oder mehrere auch markiert sein können, eine zur Verlängerung der Primer geeignete DNA-Polymerase und geeignete Puffersubstanzen. Desweiteren kann das Reagenzkit Eich- und Kontrollsubstanzen (z. B. eine Positivprobe) enthalten.

Ebenfalls Gegenstand der Erfindung ist ein Verfahren zur Amplifikation von Neisseria gonorrhoeae spezifischen Nukleotidsequenzen mit Hilfe mindestens eines Primers, wobei der Primer mit der Neisseria gonorrhoeae spezifischen Nukleotidsequenz in der 23S rRNA, insbesondere in einem Bereich hybridisieren kann, der die Positionen 1339 oder 1502 der 23S rRNA einschließt, oder der mit den entsprechenden Gegensträngen in diesen Bereichen hybridisieren kann. Auch hier gelten die genannten bevorzugten Ausführungsformen.

Ebenfalls Gegenstand der Erfindung ist ein Verfahren zur Differenzierung zwischen N. gonorrhoeae und N. meningitidis, wobei die oben genannten Primer Verwendung finden. Unter Differenzierung wird hier verstanden, daß erst die mindestens 10⁴ x größere Menge von N. meningitidis unter den gleichen Bedingungen wie N. gonorrhoeae nachgewiesen werden kann. Solche Mengen sind in üblichen biologischen Proben jedoch kaum zu erwarten. Bevorzugt liegt die Differenzierung bei einem Faktor von 10⁵ bis 10⁶.

Die folgenden Beispiele sollen die Erfindung näher erläutern:

### Beispiel 1

### Differenzierung der Neisseria gonorrhoeae Sequenz und der Neisseria meningitidis Sequenz

### 1. Verwendete Reagenzien

- BM Core Kit Plus:: [MgCl₂] 2,5 mM; [dNTP] 100 µM; [dUTP] 300 µM; [Dig-11-dUTP] 15 µM; [Primer] 200 nM; Uracil-DNA Glycosylase 2 U/Reaktion; Taq-Polymerase 2,5 U/Ansatz; Reaktionsvolumen: 100 µl

### 2. Verwendete Primer

| | |
|---|---|
| Neiss 3 (forward): 20mer, Position 1320 - 1339 | (SEQ.ID.NO. 1) |
| 5' ACG TTC ATC GGC GTA GGG AA 3' | A = zus. Basenaustausch T => A |
| Neiss 4 (reverse): 20mer, Position 1521-1502 | (SEQ.ID.NO. 2) |
| 5' CAC TTC TCG GTG TTA AGA TA 3' | T = zus. Basenaustausch A => T |
| Neiss 5 (forward): 20mer, Position 1320 - 1339 | (SEQ.ID.NO. 3) |
| 5' ACG TTC ATC GGC GTA GAG TA 3' | A = zus. Basenaustausch G => A |
| Neiss 6 (reverse): 20mer, Position 1521 - 1502 | (SEQ.ID.NO. 4) |
| 5' CAC TTC TCG GTG TTA ATA AA 3' | T = zus. Basenaustausch G => T |
| Neiss 7 (forward): 20mer; Position 1320 - 1339 | (SEQ.ID.NO. 5) |
| 5' ACG TTC ATC GGC GTA GGA TA 3' | A = zus. Basenaustausch G => A |
| Neiss 8 (reverse): 20mer; Position 1521 - 1502 | (SEQ.ID.NO. 6) |
| 5' CAC TTC TCG GTG TTA AGT AA 3' | T = zus. Basenaustausch A => T |

### 3. Vorbehandlung der Probe

Es wurden Plasmidkonstrukte verwendet, die entsprechende Sequenzen des 23S rRNA Gens von Neisseria gonorrhoeae und Neisseria meningitidis enthalten. Die Plasmide wurden in E. coli vermehrt und mit Standardprozeduren isoliert. Die DNA-Konzentration wurde durch Messung der Absorption bei 260 nm bestimmt und daraus die Anzahl der Plasmide pro ml bestimmt. Es wurden Verdünnungsreihen hergestellt, die 10¹⁰ bis 100 Plasmide pro ml enthielten. Von diesen Verdünnungsreihen wurden je 10 µl als Probe eingesetzt, so daß die PCR-Reaktionsansätze jeweils 10⁸ bis 1 Plasmid enthielten.

### 3. Amplifikation

Die oben genannte Probenlösung wurde mit den Reagenzien des BM Core Kits Plus (Boehringer Mannheim GmbH, Bestell-Nr. 1585541) in den unter 1. genannten Konzentrationen versetzt. Während der PCR-Reaktion wurde folgende Temperaturbehandlung in einem Thermocycler der Firma Perkin Elmer (Modell 9600) vorgenommen:
10 min 20 °C
10 min 94 °C
40 Zyklen (40 sec. 94 °C, 40 sec. 52 °C, 30 sec. 72 °C)
10 min 72 °C
Halten bei 72 °C

### 5. Nachweis der Amplifikate

Von den 100 µl Amplifikationsansatz werden 30 µl mit 270 µl Denaturierungslösung aus dem Enzymun-Test DNA Detektion Kit (Boehringer Mannheim GmbH, Bestell.-Nr. 1447777) versetzt und 10 min denaturiert. In einem ES 300-Analysegerät werden die Proben dann entsprechend der folgenden Testdurchführung bestimmt. Alle Inkubationen werden bei 37 °C in einem Streptavidin beschichteten Test-Tube (Boehringer Mannheim GmbH, Bestell.-Nr. 1144553) durchgeführt. Nach jeder Inkubation werden die Tubes mit der Enzymun-Test Waschlösung (Boehringer Mannheim GmbH, Bestell.-Nr. 1059475) gespült.

Zu 100 µl denaturierter Probenlösung werden 400 µl Hybridisierungspuffer gegeben, der 70 ng/ml der biotinlyierten Fangsonde (5' Biotin- GCA GAA ATG CGT AGT CGA TG 3' (SEQ.ID.NO. 7) enthält und für 2 h inkubiert. Dann werden 500 µl Konjugatpuffer zugegeben, in dem Anti-Digoxygenin POD-Konjugat (≥ 1 U/ml) in einer 1:100 Verdünnung gelöst werden und für 30 min inkubiert. Schließlich wird das Enzymun-Testsubstrat (Boehringer Mannheim GmbH, Bestell.-Nr. 1295250) zugegeben und von der POD umgesetzt. Der Substratumsatz nach 30 min wird durch Absorptionsmessung bei einer Wellenlänge von 422 nm bestimmt (ES 300, Boehringer Mannheim GmbH).

### 6. Ergebnisse

| Nachweis der Neisseria meningitidis 23S rRNA-Gensequenz | | | |
|---|---|---|---|
| Plasmide pro PCR-Reaktion: | Neiss 3/4 | Neiss 5/6 | Neiss 7/8 |
| 10 E8 | 1,313 E | 4,832 E | 5,033 E |
| 10 E8 | 1,440 E | 4,851 E | 4,964 E |
| 10 E7 | 0,096 E | 1,244 E | 3,095 E |
| 10 E7 | 0,055 E | 0,993 E | 3,556 E |
| 10 E6 | 0,170 E | 0,207 E | 0,424 E |
| 10 E6 | 0,012 E | 0,052 E | 0,494 E |
| 10 E5 | 0,007 E | 0,018 E | 0,016 E |
| 10 E5 | 0,006 E | 0,012 E | 0,018 E |
| 10 E4 | 0,012 E | 0,010 E | 0,016 E |
| 10 E4 | 0,010 E | 0,018 E | 0,012 E |
| 1000 | 0,011 E | 0,003 E | 0,013 E |
| 1000 | 0,011 E | 0,006 E | 0,009 E |
| 100 | 0,004 E | 0,000 E | 0,009 E |
| 100 | 0,017 E | 0,004 E | 0,012 E |
| 10 | 0,006 E | 0,002 E | 0,009 E |
| 10 | 0,004 E | 0,000 E | 0,010 E |
| 1 | 0,003 E | 0,003 E | 0,010 E |
| 1 | 0,002 E | 0,003 E | 0,008 E |
| Null | 0,007 E | 0,011 E | 0,012 E |

| Nachweis der Neisseria gonorrhoeae 23S rRNA-Gensequenz | | | |
|---|---|---|---|
| Plasmide pro PCR-Reaktion: | Neiss 3/4 | Neiss 5/6 | Neiss 7/8 |
| 1000 | 4,167 E | 4,715 E | 4,980 E |
| 1000 | 4,274 E | 4,734 E | 4,943 E |
| 1000 | 4,393 E | 4,750 E | 4,802 E |
| 100 | 1,007 E | 4,718 E | 2,863 E |
| 100 | 2,211 E | 4,055 E | 1,910 E |
| 100 | 0,290 E | 4,487 E | 4,439 E |
| 10 | 0,002 E | 0,004 E | 0,026 E |
| 10 | 0,004 E | 0,001 E | 0,020 E |
| 10 | 0,246 E | 2,891 E | 1,552 E |
| 1 | 0,003 E | 0,001 E | 0,022 E |
| 1 | 0,073 E | 0,005 E | 0,856 E |
| 1 | 0,726 E | 0,005 E | 0,028 E |

### 7. Auswertung

Das Primerpaar 3/4 zeigt die beste Differenzierung der N. meningitidis-Sequenz von der N. gonorrhoeae-Sequenz. Auch mit dem Primerpaar Neiss 5/6 werden erst 10⁷ Plasmide eindeutig nachgewiesen. Da die Nachweisgrenze für die N. gonorrhoeae-Sequenz für dieses Primerpaar unter 50 Zielsequenzen liegt, werden beide Sequenzen eindeutig differenziert. Die beiden Primerpaare 3/4 und 7/8 haben gegenüber dem Primerpaar Neiss 5/6 den Nachteil einer geringeren Sensitivität.

Das Experiment spiegelt die Erwartung wieder, daß die Einführung einer zusätzlichen Basenfehlpaarung möglichst nahe an das 3'-Ende der Primer die Spezifität erhöht, aber gleichzeitig die Sensitivität reduziert.

Der Basenaustausch an Position 4 vom 3'-Ende her (Neiss 5/6) stellt dabei den optimalen Kompromiß zwischen ausreichender Sensitivität und Spezifität dar.

### Beispiel 2

### Differenzierung gegenüber anderen Erregern

Die Spezifität des Primerpaares Neiss 5/6 wurde mit verschiedenen Bakterienspezies untersucht.

### Getestet wurden:

A-Steptokokken, B-Streptokokken, Staphylokokkus saprophyticus, Staphylokokkus aureus, Mycoplasmen, Ureaplasmen, Enterokokken, Proteus mirabilis, Pseudomonas aeruginosa, Gardnerella vaginalis, Escherichia coli, Chlamydia trachomatis und Neisserien aus einem Rachenabstrich (dieser enthält die Spezies N. meningitidis und andere Neisserien, nicht jedoch N. gonorrhoeae).

### 1. Probenvorbereitung

200 µl der Kultur werden hitzeinaktiviert und einmal mit Phenol/Chloroform (Verhältnis 1:1) extrahiert und einmal mit Chloroform. Dann wird die DNA mit 0,3 M Natriumacetat und 2,5 Volumen Ethanol gefällt, einmal mit 80 % Ethanol (v/v) gewaschen, getrocknet und in 200 µl Wasser rückgelöst. Von dieser Probelösung werden jeweils 20 µl für eine PCR-Amplifikation eingesetzt.

### 2. Amplifikation und Detektion

Jeweils 20 µl der Probenlösung wurden wie unter Beispiel 1 beschrieben amplifiziert und detektiert. Als Kontrolle wurden ebenfalls 1000 Plasmide mit der klonierten Neisseria gonorrhoeae 23S rRNA Gensequenz amplifiziert und detektiert.

### 3. Ergebnisse

Während die Kontrolle eine Extinktion von 4,919 E zeigte, waren die Werte für die anderen Bakterienstämme alle kleiner 0,020 E.

Im Gegensatz zu den Primern Neiss 5/6 weisen die nicht differenzierenden Primer Neiss 1 und Neiss 2 die Neisserien aus dem Rachenabstrich klar nach (mit 4,969 E).

| | |
|---|---|
| Neiss 1 (forward): 23mer, Position 1195 - 1217, | SEQ.ID.NO. 8 |
| 5' GTT CTG TAG GCT GAT GAA GGT GC 3' | |
| Neiss 2 (reverse): 24mer, Position 1528 - 1505 | SEQ.ID.NO. 9 |
| 5' TCG TCA TCA CTT CTC GGT GTT AAG 3' | |

## Patentansprüche

1. Verfahren zum Nachweis von Neisseria gonorrhoeae durch Amplifikation einer Neisseria gonorrhoeae spezifischen Nukleotidsequenz und Nachweis der Amplifikationsprodukte, dadurch gekennzeichnet, daß die Amplifikation mit Hilfe mindestens eines Primers durchgeführt wird, der mit der Neisseria gonorrhoeae spezifischen Nukleotidsequenz innerhalb der 23S rRNA hybridisieren kann, oder der mit dem entsprechenden Gegenstrang in diesem Bereich hybridisieren kann.

2. Verfahren gemäß Anspruch 1, dadurch gekennzeichnet, daß der Primer mit der N. gonorrhoeae spezifischen Nukleotidsequenz in einem Bereich hybridisieren kann, der Position 1339 der 23S rRNA einschließt, oder der mit dem entsprechenden Gegenstrang in diesem Bereich hybridisieren kann.

3. Verfahren gemäß Anspruch 1 oder 2, dadurch gekennzeichnet, daß zur Amplifikation zusätzlich ein zweiter Primer verwendet wird, der mit dem Gegenstrang in einem Bereich hybridisieren kann, der Position 1502 der 23S rRNA einschließt, oder der mit der Neisseria gonorrhoeae spezifischen Nukleotidsequenz in diesem Bereich hybridisieren kann.

4. Verfahren gemäß Anspruch 1, dadurch gekennzeichnet, daß die 3'-terminale Base des Primers komplementär oder homolog ist zu der Base in Position 1339 der 23S rRNA.

5. Verfahren gemäß Anspruch 2, dadurch gekennzeichnet, daß die 3'-terminale Base des Primers komplementär oder homolog ist zu der Base in Position 1502 der 23S rRNA.

6. Verfahren gemäß einem der Ansprüche 1 oder 2, dadurch gekennzeichnet, daß der Primer in einem 2 bis evtl. 2 bis 8 Basen vom 3'-Ende des Primers entfernten Bereich ein oder mehrere Mismatches zu der spezifischen Nukleotidsequenz oder dem Gegenstrang aufweist.

7. Verfahren gemäß Anspruch 5, dadurch gekennzeichnet, daß der Mismatch in Position 4 vom 3'-Ende des Primers lokalisiert ist.

8. Set von mindestens 2 Primern, welche eine Nukleotidsequenz aufweisen, die eine spezifische Hybridisierung
- des ersten Primers mit einer Neisseria gonorrhoeae spezifischen Nukleotidsequenz und
- des zweiten Primers mit einem Gegenstrang erlauben,
dadurch gekennzeichnet, daß einer der Primer in einem Bereich hybridisiert, der Position 1339 der 23S rRNA von Neisseria gonorrhoeae einschließt.

9. Set gemäß Anspruch 8, dadurch gekennzeichnet, daß der andere Primer in einem Bereich hybridisiert, der Position 1502 der 23S rRNA einschließt.

10. Verfahren zur Amplifikation von Neisseria gonorrhoeae spezifischen Nukleotidsequenzen mit Hilfe mindestens eines Primers, dadurch gekennzeichnet, daß der Primer mit der Neisseria gonorrhoeae spezifischen Nukleotidsequenz in einem Bereich hybridisieren kann, der die Position 1339 oder 1502 der 23S rnRNA einschließt oder mit dem entsprechenden Gegenstrang in diesem Bereich hybridisieren kann.


Sequenzprotokoll

(1) ALLGEMEINE ANGABEN:
(i) ANMELDER:
(A) NAME: BOEHRINGER MANNHEIM GMBH
(B) STRASSE: Sandhoferstr.116
(C) ORT: Mannheim
(E) LAND: DE
(F) POSTLEITZAHL: 68305
(G) TELEFON: 0621 759 4348
(H) TELEFAX: 0621 759 4457

(ii) BEZEICHNUNG DER ERFINDUNG: Verfahren zur Amplifikation von Neisseria
gonorhoeae Nukleinsauresequenzen

(iii) ANZAHL DER SEQUENZEN: 9
(iv) COMPUTER-LESBARE FASSUNG:
(A) DATENTRÄGER: Floppy disk
(B) COMPUTER: IBM PC compatible
(C) BETRIEBSSYSTEM: PC-DOS/MS-DOS
(D) SOFTWARE: PatentIn Release #1.0, Version #1.30 (EPA)

(2) ANGABEN ZU SEQ ID NO: 1:

(i) SEQUENZKENNZEICHEN:
(A) LÄNGE: 20 Basenpaare
(B) ART: Nucleotid
(C) STRANGFORM: Einzelstrang
(D) TOPOLOGIE: linear

(ii) ART DES MOLEKÜLS: Sonstige Nucleinsäure

(A) BESCHREIBUNG: /desc = "Oligodeoxyribonukleotid"

(iii) HYPOTHETISCH: NEIN

(xi) SEQUENZBESCHREIBUNG: SEQ ID NO: 1:

ACGTTCATCG GCGTAGGGAA 20

(2) ANGABEN ZU SEQ ID NO: 2:

(i) SEQUENZKENNZEICHEN:
(A) LÄNGE: 20 Basenpaare
(B) ART: Nucleotid
(C) STRANGFORM: Einzelstrang
(D) TOPOLOGIE: linear

(ii) ART DES MOLEKÜLS: Sonstige Nucleinsäure
(A) BESCHREIBUNG: /desc = "Oligodeoxyribonukleotid"

(iii) HYPOTHETISCH: NEIN

(xi) SEQUENZBESCHREIBUNG: SEQ ID NO: 2:

CACTTCTCGG TGTTAAGATA 20

(2) ANGABEN ZU SEQ ID NO: 3:

(i) SEQUENZKENNZEICHEN:
(A) LÄNGE: 20 Basenpaare
(B) ART: Nucleotid
(C) STRANGFORM: Einzelstrang
(D) TOPOLOGIE: linear

(ii) ART DES MOLEKÜLS: Sonstige Nucleinsäure
(A) BESCHREIBUNG: /desc = "Oligodeoxyribonukleotid"

(iii) HYPOTHETISCH: NEIN

(xi) SEQUENZBESCHREIBUNG: SEQ ID NO: 3:

ACGTTCATCG GCGTAGAGTA 20

(2) ANGABEN ZU SEQ ID NO: 4:

(i) SEQUENZKENNZEICHEN:
(A) LÄNGE: 20 Basenpaare
(B) ART: Nucleotid
(C) STRANGFORM: Einzelstrang
(D) TOPOLOGIE: linear

(ii) ART DES MOLEKÜLS: Sonstige Nucleinsäure
(A) BESCHREIBUNG: /desc = "Oligodeoxyribonukleotid"

(iii) HYPOTHETISCH: NEIN

(xi) SEQUENZBESCHREIBUNG: SEQ ID NO: 4:

CACTTCTCGG TGTTAATAAA 20

(2) ANGABEN ZU SEQ ID NO: 5:

(i) SEQUENZKENNZEICHEN:
(A) LÄNGE: 20 Basenpaare
(B) ART: Nucleotid

(C) STRANGFORM: Einzelstrang
(D) TOPOLOGIE: linear

(ii) ART DES MOLEKÜLS: Sonstige Nucleinsäure
(A) BESCHREIBUNG: /desc = "Oligodeoxyribonukleotid"

(iii) HYPOTHETISCH: NEIN

(xi) SEQUENZBESCHREIBUNG: SEQ ID NO: 5:

ACGTTCATCG GCGTAGGATA 20

(2) ANGABEN ZU SEQ ID NO: 6:

(i) SEQUENZKENNZEICHEN:
(A) LÄNGE: 20 Basenpaare
(B) ART: Nucleotid
(C) STRANGFORM: Einzelstrang
(D) TOPOLOGIE: linear

(ii) ART DES MOLEKÜLS: Sonstige Nucleinsäure
(A) BESCHREIBUNG: /desc = "Oligodeoxyribonukleotid"

(iii) HYPOTHETISCH: NEIN

(xi) SEQUENZBESCHREIBUNG: SEQ ID NO: 6:

CACTTCTCGG TGTTAAGTAA 20

(2) ANGABEN ZU SEQ ID NO: 7:

(i) SEQUENZKENNZEICHEN:

(A) LÄNGE: 20 Basenpaare
(B) ART: Nucleotid
(C) STRANGFORM: Einzelstrang
(D) TOPOLOGIE: linear

(ii) ART DES MOLEKÜLS: Sonstige Nucleinsäure
(A) BESCHREIBUNG: /desc = "Oligodeoxyribonukleotid"

(iii) HYPOTHETISCH: NEIN

(ix) MERKMAL:
(A) NAME/SCHLÜSSEL: misc_feature
(B) LAGE:1
(D) SONSTIGE ANGABEN:/note= "Am 5'-Phosphatende mit Biotin ueber
Aminomodifier markiert"

(xi) SEQUENZBESCHREIBUNG: SEQ ID NO: 7:

GCAGAAATGC GTAGTCGATG 20

(2) ANGABEN ZU SEQ ID NO: 8:

(i) SEQUENZKENNZEICHEN:
(A) LÄNGE: 23 Basenpaare
(B) ART: Nucleotid
(C) STRANGFORM: Einzelstrang
(D) TOPOLOGIE: linear

(ii) ART DES MOLEKÜLS: Sonstige Nucleinsäure
(A) BESCHREIBUNG: /desc = "Oligodeoxyribonukleotid"

(iii) HYPOTHETISCH: NEIN

(xi) SEQUENZBESCHREIBUNG: SEQ ID NO: 8:

GTTCTGTAGG CTGATGAAGG TGC 23

(2) ANGABEN ZU SEQ ID NO: 9:

(i) SEQUENZKENNZEICHEN:
(A) LÄNGE: 24 Basenpaare
(B) ART: Nucleotid
(C) STRANGFORM: Einzelstrang
(D) TOPOLOGIE: linear

(ii) ART DES MOLEKÜLS: Sonstige Nucleinsäure
(A) BESCHREIBUNG: /desc = "Oligodeoxyribonukleotid"

(iii) HYPOTHETISCH: NEIN

(xi) SEQUENZBESCHREIBUNG: SEQ ID NO: 9:

TCGTCATCAC TTCTCGGTGT TAAG 24